# EUROPEAN PATENT APPLICATION

(11) **EP 4 404 168 A1**
(43) Date of publication of application: **24.07.2024**
(21) Application number: 23151909.1
(22) Date of filing: 17.01.2023
(51) Int. Cl.: G08G 1/005, G08G 1/0965, G08G 1/00, G06V 20/58, G16H 40/67, H04W 4/90, G08B 25/01, H04W 4/44

(54) **METHOD FOR CLEARING A TRAFFIC PARTICIPANT INCIDENT, DATA PROCESSING APPARATUS, VEHICLE, COMPUTER PROGRAM, AND COMPUTER-READABLE STORAGE MEDIUM**

(71) Applicant: Volvo Car Corporation, 405 31 Göteborg (SE)
(72) Inventor: MORALES, Gerardo, 40531 Göteborg (SE); PEREZ BARRERA, Oswaldo, 40531 Göteborg (SE)
(74) Representative: Maiwald GmbH

(57) **Abstract**

The disclosure relates to a method for clearing a traffic participant incident occurring in a proximity of a vehicle (10) comprising an environment detection sensor (26). The method comprises receiving an incident notification (IN) from an electronic device (36) of the traffic participant (38) having the incident or from an electronic device of a witnessing traffic participant. The incident notification (IN) comprises a location information (LI) describing the location of the traffic participant (38). Moreover, the method comprises activating the environment detection sensor (26). Additionally, the method comprises receiving an environment detection result (DR) from the environment detection sensor (26), wherein the environment detection result (DR) describes an environment of the vehicle (10). The method also comprises providing the received environment detection result (DR) to an emergency service (34). Furthermore, a data processing apparatus (14), a vehicle (10), a computer program (22), and a computer-readable storage medium (20) are presented.

## Description

The present disclosure relates to a method for clearing a traffic participant incident.

The present disclosure further is directed to a corresponding data processing apparatus, a vehicle, a corresponding computer program, and a corresponding computer-readable storage medium.

When participating in traffic, a traffic participant may be subject to an undesired incident. The incident may involve a threat to the traffic participant's soundness. More generally speaking, such an incident reduces a level of safety and/or security of the traffic participant and of a traffic system comprising the traffic participant.

The incident may be a medical emergency. The medical emergency may for example result from an accident in which the traffic participant is involved. Additionally or alternatively, the incident may relate to the fact that the traffic participant is a victim of a criminal act.

It is, therefore, an objective of the present disclosure to increase the safety and security of a traffic participant and a traffic system comprising the traffic participant.

The problem is at least partially solved or alleviated by the subject matter of the independent claims of the present disclosure, wherein further examples are incorporated in the dependent claims.

According to a first aspect, there is provided a method for clearing a traffic participant incident. The traffic participant incident occurs in a proximity of a vehicle comprising an environment detection sensor. The method comprises:
- receiving an incident notification from an electronic device of the traffic participant having the incident or from an electronic device of a witnessing traffic participant, wherein the incident notification comprises a location information describing the location of the traffic participant,
- activating the environment detection sensor,
- receiving an environment detection result from the environment detection sensor, wherein the environment detection result describes an environment of the vehicle, and
- providing the received environment detection result to an emergency service.

This method may be executed by a component of the vehicle. In this context, a witnessing traffic participant is a traffic participant observing the traffic participant incident. As has been mentioned before, the traffic participant incident relates to a situation of a traffic participant which threatens the traffic participant's soundness. The incident may be a medical emergency. Additionally or alternatively, the incident may relate to the fact that the traffic participant is a victim of a criminal act. Consequently, the incident notification is to be understood as a notification that the corresponding traffic participant is in a situation in which his or her soundness is threatened. The electronic device may be a portable electronic device such as a smart phone, a tablet, a smart watch or any other portable or wearable electronic device. Electronic devices being able to send an incident notification are generally known. According to the present method, such an incident notification is received at a vehicle being located close to the location at which the incident is happening. In an example, the proximity of the vehicle may be defined by the detection range of the environment detection sensor of the vehicle. By activating the environment detection sensor and receiving an environment detection result from the environment detection sensor, valuable information describing the incident may be collected by the vehicle. Once provided to an emergency service, this information may be useful in order to help the traffic participant subject to the incident. Based on the environment detection result, appropriate help may be provided in a quick manner. It is noted that the environment detection result may be provided to the emergency service in a direct or indirect manner. The indirect manner may involve routing the environment detection result by an intermediary. Altogether, the incident may be cleared in a timely and efficient manner. This means that the soundness of the traffic participant being subject to the incident is reinstated or the magnitude of the threat to the soundness of the traffic participant is at least reduced.

It is noted that the vehicle on which the method of the present disclosure is run is not involved in the incident. The vehicle on which the method of the present disclosure is performed may be moving or standing still, e.g. parked, while performing the present method. Moreover, the vehicle may be unoccupied and/or locked.

It is further noted that the present method does not exclude that the same or a different incident notification is transmitted from the electronic device of the traffic participant to the emergency service in a direct or indirect manner without passing by the vehicle. In this context, the environment detection result provided by the vehicle and the same or different incident notification provided by the electronic device of the traffic participant may be matched by the emergency service. To this end, the incident notification may comprise an identifier which may be a notification identifier or an identifier of the electronic device from which the incident notification is sent.

The emergency service may be a hospital or clinic, a fire department, a police department or any other authority having the capability and authority to help clearing the incident.

The traffic participant having the incident is for example a pedestrian, a cyclist or a rider of a motorcycle. Such traffic participants may be summarized as vulnerable road users.

In an example, the incident notification is generated by a fall detection application of the electronic device. In another example, the incident notification is generated by a health tracking application of the electronic device. In a further example, the incident notification is generated by a safety application of the electronic device.

In an example, the environment detection sensor comprises a camera. The environment detection result comprises at least one image. This image potentially shows at least a part of the incident and is provided to the emergency service. Thus, the emergency service can react to the incident based on the at least one image. The reaction may therefore be appropriate and timely.

In an example, the method further comprises performing an object recognition technique on the received environment detection result, generating a marking highlighting a representation of the recognized object in the environment detection result, and providing the received environment detection result with the marking to the emergency service. In a case in which the environment detection result comprises at least one image, the object recognition technique is performed on the at least one image. In doing so, the traffic participant being subject to the incident and/or other traffic participants may be detected and marked in the environment detection result. Based thereon, a number of traffic participants being involved in the incident may be estimated. Additionally or alternatively, performing the object recognition technique may be used to detect further features or characteristics of the incident, such as big blood losses. As has been mentioned before, the traffic participant being subject to the incident may be a pedestrian, a cyclist or a rider of a motorcycle of any kind. Due to the marking, the emergency service may derive characteristics of the incident in a quick and reliable manner.

In an example, the method further comprises estimating a distance between the vehicle and the recognized object and providing the estimated distance to the emergency service. This information further helps the emergency service to clear the incident in an efficient and appropriate manner.

In an example, the method further comprises mapping the location information on the environment detection result, generating a location identifier highlighting the portion of the environment detection result representing the location, and providing the received environment detection result with the location identifier to the emergency service. In other words, the location identifier highlights the portion of the environment detection result, e.g. the portion of an image, which shows the traffic participant being subject to the incident. The emergency service can, thus, quickly and reliably analyze the environment detection result and trigger appropriate measures.

In an example, the method further comprises providing an incident information to the emergency service. In this context, an incident information is an information describing the incident or the context in which the incident has happened. The incident information for example comprises at least one of an name of the user of the electronic device, and age of the user of the electronic device, a gender of the user of the electronic device, an identifier of the electronic device, and intensity of the incident having been measured by the electronic device and a vitality indicator such as a heart rate having been measured or received by the electronic device. Thus, the incident information helps the emergency service to trigger an appropriate reaction to the incident.

It is noted that the fact that the incident information is provided to the emergency service via the vehicle using the present method does not exclude that the same or different incident information is provided to the emergency service by the electronic device in a direct or indirect manner without passing by the vehicle.

In an example, the method further comprises determining whether activating the environment detection sensor is authorized and activating the environment detection sensor only if authorized. In a case in which activating the environment detection sensor is authorized, the present method is continued. Otherwise, i.e. if activating the environment detection sensor is not authorized, the method is abandoned. Thus, the performance of the present method can be selectively enabled or disabled.

In an example, the method further comprises determining a distance between the location described by the location information and a location of the vehicle, and abandoning the method if the distance exceeds a predefined distance threshold. In simplified words, the method is only performed if the vehicle is located sufficiently close to the location of the incident. This ensures that the environment detection sensor is able to at least partially detect the incident and/or surroundings of the incident with sufficient detail.

In an example, the incident notification is an automatic notification generated by the electronic device. Alternatively, the incident notification is a user generated notification resulting from an action of a user of the electronic device. The incident notification may be an automatic notification in a case in which it is generated by a fall detection application running on the electronic device or in a case in which it is generated by a health tracking application running on the electronic device. A user generated notification may be generated by the user, i.e. the traffic participant subject to the incident or a witnessing traffic participant by pressing a button. In all of the above cases, the incident notification is generated in a reliable manner.

In an example, the method further comprises evaluating the presence of another vehicle closer to the location being described by the location information. The environment detection sensor of a vehicle being closer to the location being described by the location information, i.e. the location of the incident, may be able to provide an environment detection result which shows the incident or an environment of the incident in more detail. Based thereon, the emergency service may be in a position to derive a more appropriate reaction to the incident. Thus, environment detection results of high quality may be generated and provided to the emergency service.

The method may be at least partly computer-implemented, and may be implemented in software or in hardware, or in software and hardware. Further, the method may be carried out by computer program instructions running on means that provide data processing functions. The data processing means may be a suitable computing means, such as an electronic control module etc., which may also be a distributed computer system. The data processing means or the computer, respectively, may comprise one or more of a processor, a memory, a data interface, or the like.

It is noted that it is of course also possible that the method of the present disclosure is performed by two or more vehicles at the same time or at different times but relating to the same incident notification. Thus, environment detection results originating from different vehicles may be received at the emergency service. Consequently, the emergency service has detailed information on the incident and is able to trigger an appropriate reaction thereto.

According to a second aspect, there is provided a data processing apparatus comprising means for carrying out the method of the present disclosure. Thus, valuable information describing the incident may be collected and provided to an emergency service. This information may be useful in order to help the traffic participant subject to the incident. Due to the environment detection result, appropriate help may be provided in a quick manner.

According to a third aspect, there is provided a vehicle comprising a data processing apparatus according to the present disclosure. Using such a vehicle, valuable information describing the incident may be collected and provided to an emergency service. This information may be useful in order to help the traffic participant subject to the incident. Due to the environment detection result, appropriate help may be provided in a quick manner. It is noted that the vehicle may be able to provide this information independent from the fact that the vehicle is occupied or not.

In an example, the vehicle comprises an environment detection sensor and the environment detection sensor is communicatively connected to the data processing apparatus. Consequently, an environment detection result having been generated by the environment detection sensor can be provided to the data processing apparatus in a reliable manner.

In an example, the vehicle comprises a communication unit being communicatively connected to the data processing apparatus. The communication unit may be configured to exchange data with the emergency service and/or the electronic device of the traffic participant being subject to the incident. Thus, an incident notification may be reliably received and/or the environment detection result may be reliably provided to the emergency service.

According to a fourth aspect, there is provided a computer program comprising instructions which, when the computer program is executed by a computer, cause the computer to carry out the method of the present disclosure. Thus, valuable information describing the incident may be collected and provided to an emergency service. This information may be useful in order to help the traffic participant subject to the incident. Due to the environment detection result, appropriate help may be provided in a quick manner.

According to a fifth aspect, there is provided a computer-readable storage medium comprising instructions which, when executed by a computer, cause the computer to carry out the method of the present disclosure. Thus, valuable information describing the incident may be collected and provided to an emergency service. This information may be useful in order to help the traffic participant subject to the incident. Due to the environment detection result, appropriate help may be provided in a quick manner.

It should be noted that the above examples may be combined with each other irrespective of the aspect involved.

These and other aspects of the present disclosure will become apparent from and elucidated with reference to the examples described hereinafter.

Examples of the disclosure will be described in the following with reference to the following drawings.

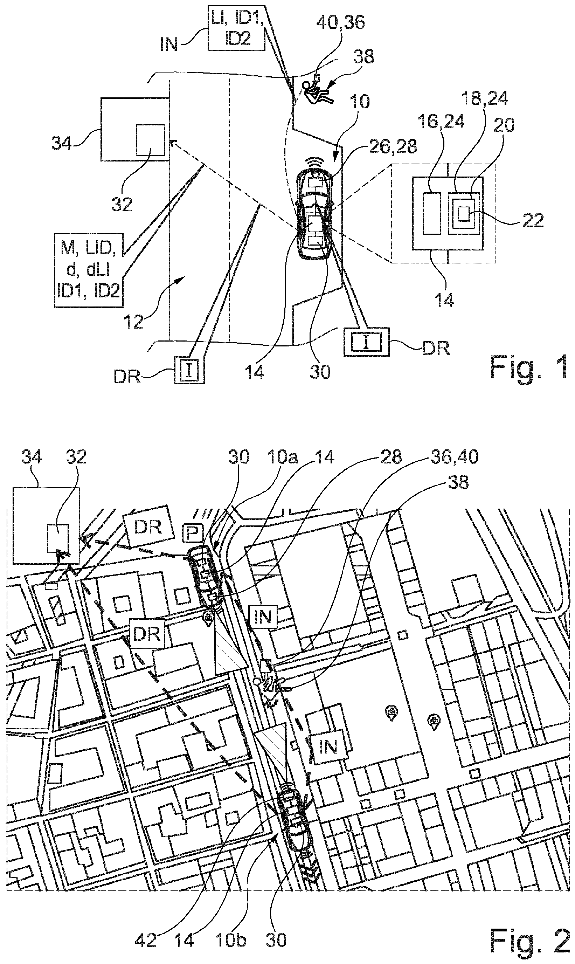

- Figure 1: shows a first traffic situation comprising a vehicle according to the present disclosure having a data processing apparatus according to the present disclosure, a computer-readable storage medium according to the present disclosure, and a computer program according to the present disclosure, wherein a method according to the present disclosure may be performed on the vehicle, and a traffic participant being subject to an incident,
- Figure 2: shows a second traffic situation comprising two vehicles according to the present disclosure, each having a data processing apparatus according to the present disclosure, a computer-readable storage medium according to the present disclosure, and a computer program according to the present disclosure, wherein a method according to the present disclosure may be performed on each of the vehicles, and a traffic participant being subject to an incident, and

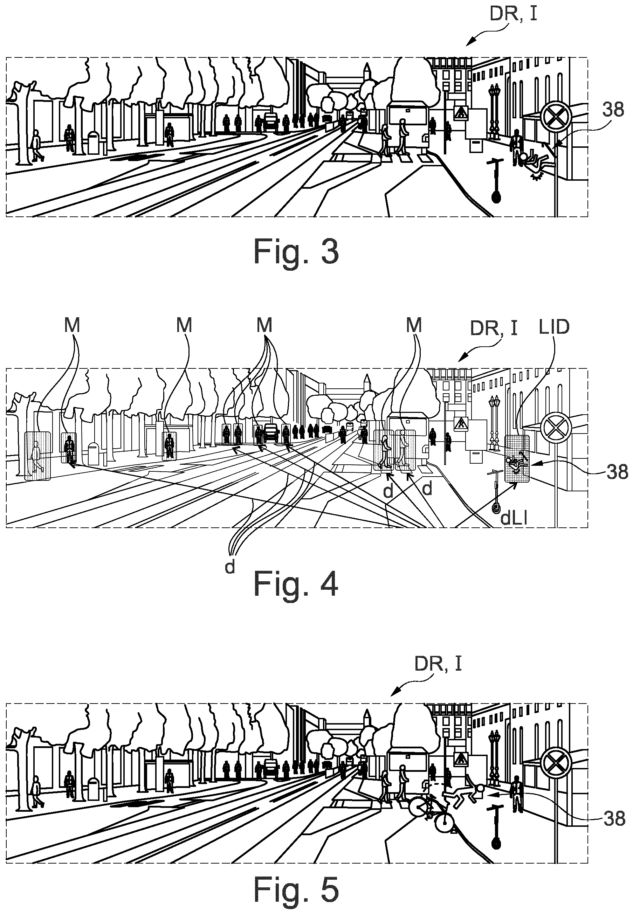

- Figures 3 to 5: show examples of environment detection results of the vehicle of Figure 1 or the vehicles of Figure 2.

The Figures are merely schematic representations and serve only to illustrate examples of the disclosure. Identical or equivalent elements are in principle provided with the same reference signs.

Figure 1 shows a first traffic situation comprising a vehicle 10. The vehicle 10 is parked at the side of a road 12. Thus, the vehicle 10 is standing still. Moreover, the vehicle 10 is un-occupied and locked.

The vehicle 10 comprises a data processing apparatus 14.

The data processing apparatus 14 has a data processing unit 16 and a data storage unit 18.

The data storage unit 18, comprises a computer-readable storage medium 20.

On the computer-readable storage medium 20, there is provided a computer program 22.

The computer-readable storage medium 20 and the computer program 22 comprise instructions which, when executed by the data processing unit 16 or, more generally speaking, a computer, cause the data processing unit 26 or the computer to carry out a method for clearing a traffic participant incident.

Consequently, the data processing unit 16 and the data storage unit 18 form means 24 for carrying out the method for clearing a traffic participant incident.

The vehicle 10 additionally comprises an environment detection sensor 26 which in the present example is a camera 28, more precisely a frontal camera.

The environment detection sensor 26, i.e. the camera 28 is communicatively connected to the data processing apparatus 14.

Moreover, the vehicle 10 comprises a communication unit 30 being configured to exchange data with a data processing apparatus 32 of an emergency service 34 which also forms part of the traffic situation.

In the present example, the emergency service 34 is an ambulance service.

Beyond that, the communication unit 30 is configured to exchange data with an electronic device 36 as will be explained further below.

The communication unit 30 is communicatively connected to the data processing apparatus 14.

The first traffic situation additionally comprises a traffic participant 38 being subject to an incident. In the present example, the traffic participant 38 is a pedestrian who has fallen.

The traffic participant 38 carries the electronic device 36 which in the present example is a smart phone 40.

The electronic device 36, i.e. the smart phone 40, is communicatively connected to the communication unit 30.

As has been mentioned before, in the present traffic situation the traffic participant 38 has fallen. This circumstance is detected by a fall detection application running on the electronic device 36. As a consequence thereof, the electric device 36 sends out and incident notification IN.

Thus, in the present example, the incident notification IN is an automatic notification generated by the electronic device 36. In an alternative, the incident notification IN could be a user generated notification resulting from an action of a user of the electronic device 36, e.g. the traffic participant 38 or a witnessing traffic participant.

At the same time, the method for clearing a traffic participant incident is performed on the data processing apparatus 14 of the vehicle 10.

It is noted that the incident has occurred in a proximity of the vehicle 10.

In a first step S1, the incident notification IN is received at the data processing apparatus 14 via the communication unit 30.

In the present example, the incident notification comprises a location information LI describing the location of the traffic participant 38, an identifier ID1 characterizing the user of the electronic device 36 which in the present example is identical to the traffic participant 38 and an identifier ID2 characterizing the electronic device 36. The identifier ID1 and the identifier ID2 may be summarized as incident information II.

In an optional second step S2, it is evaluated whether other vehicles are located closer to the location being described by the location information LI than the vehicle 10. In the present example, this is not the case.

In a third step S3, the method determines whether activating the environment detection sensor 26 is authorized.

In the present example, activating the environment detection sensor 26 is authorized. In the opposite case, the method would be abandoned.

Subsequently, in a fourth step S4, the environment detection sensor 26 is activated.

This means that the environment detection sensor 26 may generate an environment detection result DR by performing an environment detection activity.

Since in the present example, the environment detection sensor 26 comprises a camera 28, performing an environment detection activity means capturing one or more images I.

Thereafter, in a fifth step S5, the environment detection result DR, i.e. the image I, is received at the data processing apparatus 14.

In the present example, the incident has happened in the field of view of the camera 28. Consequently, the environment detection result DR describes an environment of the vehicle 10 and also comprises a representation of the traffic participant 38 being subject to the incident.

Subsequently, in a sixth step S6, an object recognition technique is performed on the received environment detection result DR, i.e. on the image I.

In the present example of the object recognition technique relates to the recognition of pedestrians. For each recognized pedestrian, a marking M highlighting a representation of the recognized pedestrian is generated in the environment detection result DR (see also Figure 4).

Moreover, in a seventh step S7, a distance d between the vehicle 10 and all of the recognized objects, i.e. all of the recognized pedestrians is estimated. For the ease of representation, only some distances d are shown in Figure 4.

Additionally, in an eighth step S8, a distance dLI between the location described by the location information LI and a location of the vehicle 10 is estimated. The method is only continued if this distance dLI does not exceed a predefined distance threshold.

Moreover, in a ninth step S9, the location information LI is mapped on the environment detection result DR, i.e. the image I, and a location identifier LID highlighting the portion of the environment detection result DR representing the location is generated. To this end, also the estimated distances as explained above may be used (see also Figure 4).

Thereafter, in a tenth step S 10, the environment detection result DR is provided to the emergency service 34 together with the marking M and the location identifier LID. Also the estimated distances d of all detected objects may be provided to the emergency service 34.

Beyond that, also the identifier ID1 characterizing the user of the electronic device 36 and the identifier ID2 characterizing the electronic device 36 are provided to the emergency service 34.

Figure 2 shows a second traffic situation. This traffic situation comprises two vehicles of the kind as described in connection with Figure 1. In order to be able to distinguish these vehicles, they are denoted vehicle 10a and vehicle 10b. In the following, only the differences with respect to the first traffic situation of Figure 1 will be described. Beyond that, reference is made to the above explanations which have been provided in connection with Figure 1.

As before, traffic participant 38 who is a pedestrian has fallen.

However, in contrast to the traffic situation of Figure 1, now vehicle 10a is standing still and vehicle 10b is moving.

The incident notification IN is received by both the vehicles 10a, 10b.

When evaluating the presence of another vehicle closer to the location being described by the location information LI at a first point in time, it is found that the vehicle 10b is located closer to the location being described by the location information LI. Consequently, a detection result being generated by the environment detection sensor 26 of vehicle 10b is provided to the emergency service 34.

In the example of Figure 2, the environment detection sensor 26 of the vehicle 10b is a camera 42, more precisely a rear-facing camera.

However, the vehicle 10b is traveling away from the location being described by the location information LI.

Thus, when evaluating the presence of another vehicle closer to the location being described by the location information LI at a second point in time which is later than the first point in time, it is found that the vehicle 10a is located closer to the location being described by the location information LI.

Consequently, starting from the second point in time, a detection result DR being generated by the environment detection sensor 26 of the vehicle 10a is provided to the emergency service 34.

Figures 3 to 5 show exemplary detection results DR.

Figure 3 shows an image I comprising a representation of the traffic participant 38 who has fallen.

Figure 4 shows an image I on which an object recognition technique has been performed. In the image, all pedestrians have been highlighted using a marking M.

Moreover, the image I of Figure 4 comprises a location identifier LID highlighting the portion of the image I representing the location corresponding to the location information LI of the incident notification, i.e. the location from which the incident notification was issued.

Figure 5 shows an image I of an alternative incident. In the case of Figure 5 the traffic participant 38 has fallen off his or her bike, i.e. the traffic participant 38 had a bike accident.

Other variations to the disclosed examples can be understood and effected by those skilled in the art in practicing the claimed disclosure, from the study of the drawings, the disclosure, and the appended claims. In the claims the word "comprising" does not exclude other elements or steps and the indefinite article "a" or "an" does not exclude a plurality. A single processor or other unit may fulfill the functions of several items or steps recited in the claims. The mere fact that certain measures are recited in mutually different dependent claims does not indicate that a combination of these measures cannot be used to advantage. A computer program may be stored/distributed on a suitable medium such as an optical storage medium or a solid-state medium supplied together with or as part of other hardware, but may also be distributed in other forms, such as via the Internet or other wired or wireless telecommunication systems. Any reference signs in the claims should not be construed as limiting the scope of the claims.

### LIST OF REFERENCE SIGNS

- 10: vehicle
- 10a: vehicle
- 10b: vehicle
- 12: road
- 14: data processing apparatus of the vehicle
- 16: data processing unit
- 18: data storage unit
- 20: computer-readable storage medium
- 22: computer program
- 24: means for carrying out a method for clearing a traffic participant incident
- 26: environment detection sensor
- 28: camera
- 30: communication unit
- 32: data processing apparatus of an emergency service
- 34: emergency service
- 36: electronic device
- 38: traffic participant
- 40: smart phone
- 42: camera

- d: distance between the vehicle and a recognized object
- dLI: distance between the location described by the location information and the vehicle
- DR: environment detection result
- I: image
- II: incident information
- ID1: identifier characterizing the user of the electronic device
- ID2: identifier characterizing the electronic device
- IN: incident notification
- LID: location identifier
- M: marking
- S1: first step
- S2: second step
- S3: third step
- S4: fourth step
- S5: fifth step
- S6: sixth step
- S7: seventh step
- S8: eighth step
- S9: ninth step
- S10: tenth step

## Claims

1. A method for clearing a traffic participant incident occurring in a proximity of a vehicle (10, 10a, 10b) comprising an environment detection sensor (26), comprising:
- receiving an incident notification (IN) from an electronic device (36) of the traffic participant (38) having the incident or from an electronic device of a witnessing traffic participant, wherein the incident notification (IN) comprises a location information (LI) describing the location of the traffic participant (38),
- activating the environment detection sensor (26),
- receiving an environment detection result (DR) from the environment detection sensor (26), wherein the environment detection result (DR) describes an environment of the vehicle (10, 10a, 10b), and
- providing the received environment detection result (DR) to an emergency service (34).

2. The method of claim 1, wherein the environment detection sensor (26) comprises a camera (28, 42) and wherein the environment detection result (DR) comprises at least one image (I).

3. The method of claim 1 or 2, further comprising performing an object recognition technique on the received environment detection result (DR), generating a marking (M) highlighting a representation of the recognized object in the environment detection result (DR), and providing the received environment detection result (DR) with the marking (M) to the emergency service (34).

4. The method of claim 3, further comprising estimating a distance (d) between the vehicle (10, 10a, 10b) and the recognized object and providing the estimated distance (d) to the emergency service (34).

5. The method of any one of the preceding claims, further comprising mapping the location information (LI) on the environment detection result (DR), generating a location identifier (LID) highlighting the portion of the environment detection result (DR) representing the location, and providing the received environment detection result (DR) with the location identifier (LID) to the emergency service (34).

6. The method of any one of the preceding claims, further comprising providing an incident information (II) to the emergency service (34).

7. The method of any one of the preceding claims, further comprising determining whether activating the environment detection sensor (26) is authorized and activating the environment detection sensor (26) only if authorized.

8. The method of any one of the preceding claims, further comprising determining a distance (dLI) between the location described by the location information (LI) and a location of the vehicle (10, 10a, 10b), and abandoning the method if the distance (dLI) exceeds a predefined distance threshold.

9. The method of any one of the preceding claims, wherein the incident notification (IN) is an automatic notification generated by the electronic device (36) or wherein the incident notification (IN) is a user generated notification resulting from an action of a user of the electronic device (36).

10. The method of any one of the preceding claims, further comprising evaluating the presence of another vehicle (10, 10a, 10b) closer to the location being described by the location information (LI).

11. A data processing apparatus (14) comprising means (24) for carrying out the method of any one of the preceding claims.

12. A vehicle (10, 10a, 10b) comprising a data processing apparatus (14) according to claim 13.

13. A computer program (22) comprising instructions which, when the computer program (22) is executed by a computer, cause the computer to carry out the method of claims 1 to 10.

14. A computer-readable storage medium (20) comprising instructions which, when executed by a computer, cause the computer to carry out the method of claims 1 to 10.

## Amended claims

### Amended claims in accordance with Rule 137(2) EPC.

1. A method for clearing a traffic participant incident occurring in a proximity of a vehicle (10, 10a, 10b) comprising an environment detection sensor (26), comprising:
- receiving an incident notification (IN) from an electronic device (36) of the traffic participant (38) having the incident or from an electronic device of a witnessing traffic participant, wherein the incident notification (IN) comprises a location information (LI) describing the location of the traffic participant (38),
- activating the environment detection sensor (26),
- receiving an environment detection result (DR) from the environment detection sensor (26), wherein the environment detection result (DR) describes an environment of the vehicle (10, 10a, 10b), and
- providing the received environment detection result (DR) to an emergency service (34).

2. The method of claim 1, wherein the environment detection sensor (26) comprises a camera (28, 42) and wherein the environment detection result (DR) comprises at least one image (I).

3. The method of claim 1 or 2, further comprising performing an object recognition technique on the received environment detection result (DR), generating a marking (M) highlighting a representation of the recognized object in the environment detection result (DR), and providing the received environment detection result (DR) with the marking (M) to the emergency service (34).

4. The method of claim 3, further comprising estimating a distance (d) between the vehicle (10, 10a, 10b) and the recognized object and providing the estimated distance (d) to the emergency service (34).

5. The method of any one of the preceding claims, further comprising mapping the location information (LI) on the environment detection result (DR), generating a location identifier (LID) highlighting the portion of the environment detection result (DR) representing the location, and providing the received environment detection result (DR) with the location identifier (LID) to the emergency service (34).

6. The method of any one of the preceding claims, further comprising providing an incident information (II) to the emergency service (34).

7. The method of any one of the preceding claims, further comprising determining whether activating the environment detection sensor (26) is authorized and activating the environment detection sensor (26) only if authorized.

8. The method of any one of the preceding claims, further comprising determining a distance (dLI) between the location described by the location information (LI) and a location of the vehicle (10, 10a, 10b), and abandoning the method if the distance (dLI) exceeds a predefined distance threshold.

9. The method of any one of the preceding claims, wherein the incident notification (IN) is an automatic notification generated by the electronic device (36) or wherein the incident notification (IN) is a user generated notification resulting from an action of a user of the electronic device (36).

10. The method of any one of the preceding claims, further comprising evaluating the presence of another vehicle (10, 10a, 10b) closer to the location being described by the location information (LI).

11. A data processing apparatus (14) comprising means (24) for carrying out the method of any one of the preceding claims.

12. A vehicle (10, 10a, 10b) comprising a data processing apparatus (14) according to claim 11.

13. A computer program (22) comprising instructions which, when the computer program (22) is executed by a computer, cause the computer to carry out the method of claims 1 to 10.

14. A computer-readable storage medium (20) comprising instructions which, when executed by a computer, cause the computer to carry out the method of claims 1 to 10.
